Europäisches Patentamt

European Patent Office

Office européen des brevets

(19)

(11) **EP 1 530 977 A1**

(12) **EUROPEAN PATENT APPLICATION**

(43) Date of publication:
**18.05.2005 Bulletin 2005/20**

(51) Int Cl.⁷: **A61L 31/04**

(21) Application number: **04025831.1**

(22) Date of filing: **29.10.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **17.11.2003 JP 2003386751**

(71) Applicant: **Olympus Corporation**
**Tokyo 151-0072 (JP)**

(72) Inventors:
- **Tokuda, Kazunari**
  **Hachioji-shi Tokyo 193-0834 (JP)**
- **Kawano, Yoshihiro**
  **Hachioji-shi Tokyo 192-0916 (JP)**

(74) Representative: **von Hellfeld, Axel, Dr. Dipl.-Phys.**
**Wuesthoff & Wuesthoff**
**Patent- und Rechtsanwälte**
**Schweigerstrasse 2**
**81541 München (DE)**

(54) **Observation window member and experimental animal having observation window**

(57)    In order to enable observation of an experimental animal over a long term, keeping it without stress and in a healthy condition, an observation window member is provided which is made from an optically transparent and biocompatible material formed in a plate or membrane shape to be in close contact with internal tissue of an experimental animal in an area where the epidermis has been removed.

EP 1 530 977 A1

## Description

BACKGROUND OF THE INVENTION

1. FIELD OF THE INVENTION

The present invention relates to an observation window member and an experimental animal having an observation window.

Priority is claimed on Japanese Patent Application No. 2003-386751, filed November 17, 2003, the content of which is incorporated herein by reference.

2. DESCRIPTION OF RELATED ART

Recently, various experimental animals have been produced due to the development of gene recombination techniques. Particularly, diseased model mice having internal diseases have been produced by gene recombination, and used for validation in drug research.

As a method for detecting the condition inside cells of an experimental animal, for example, a detector such as a confocal microscope has been developed (refer to Japanese Unexamined Patent Application, First Publication No. Hei 11-133306 (FIG. 1) for example).

Moreover, as a method for observing the phenomena inside cells in detail, fluorescent proteins such as Green Fluorescent Protein (GFP) is introduced to inside the cells to impart fluorescent labeling to the specific intracellular structure such as carcinoma cells, and thereby enable observation of the behavior of the carcinoma cells from outside (for example, refer to the web page on the internet at URL: http://www.riken.go.jp/r-world/info/release/press/2001/011229/ "Press Release: Development of new fluorescent protein that enables the detailed study of live cells; Unknown phenomena inside cells are now visible." by Atsushi Miyawaki et. al. as of December 29, 2001, the Institute of Physical and Chemical Research RIKEN (searched on October 30, 2003))

However, when an attempt is made to observe carcinoma cells inside the body of an experimental animal such as a diseased model mouse, the fluorescence is scattered by the epidermis, inconveniently interfering with the observation of the image in high resolution. On the other hand, although it is possible to bring a microscope close to the internal tissue, from which the epidermis was peeled off, for observation, the experimental animal is overstressed if the observation takes a long time. Therefore, there has been a problem in that it is difficult to carry out long term observation of an experimental animal while keeping it in a healthy condition.

BRIEF SUMMARY OF THE INVENTION

The present invention takes the above problems into consideration with an object of providing an observation window member which enables observation of an experimental animal over a long term, keeping it without stress and in a healthy condition, and an experimental animal constituted therewith.

In order to solve the above problem, the present invention provides the solutions described below. An aspect of the present invention is an observation window member made from an optically transparent and biocompatible material formed in a plate or membrane shape to be in close contact with internal tissue of an experimental animal in an area where the epidermis has been removed.

According to this aspect of the present invention, the transparent observation window member in a plate or membrane shape is brought into close contact with the internal tissue, enabling observation of the internal tissue through the observation window member from the outside. In this case, since the observation window member is made from a biocompatible material, the internal tissue can be kept in a healthy condition. Therefore, the internal tissue of the experimental animal can be stably observed over a long term.

In the above aspect of the invention, preferably the observation window member is made from a flexible material.

According to this structure of the invention, the observation window member can be curved to fit with the internal tissue due to the flexibility, so as to form an observation window which can be in closer contact with the internal tissue. By increasing the closeness of contact with the internal tissue, the internal tissue can be kept from deterioration due to foreign substances, and the visibility of the internal tissue from the outside can be increased.

In the above aspect of the invention, preferably the observation window member has an attachment section for the experimental animal.

According to this structure of the invention, the observation window member can be easily attached to the experimental animal using the attachment section. Moreover, even if the experimental animal moves, the attached state is retained, enabling observation over a long term.

In the above aspect of the invention, preferably the attachment section is made of a plurality of through holes which are provided at intervals along the periphery and through which a thread for stitching to the epidermis of the experimental animal can pass.

By using the through holes to pass the thread therethrough to stitch the observation window member to the epidermis, the observation window member can be attached to the experimental animal more easily and reliably.

In the above aspect of the invention, the attachment section may comprise an adhesive applied on one face.

By bringing the face applied with the adhesive into close contact with the internal tissue, the observation window member can be adhered to the internal tissue. By employing an optically transparent adhesive as the adhesive, the observation window member can be easily attached to the experimental animal without impairing visibility from the outside. Preferably a material having an intermediate refractive index between the refractive index of the material constituting the observation window member and the refractive index of water is employed for the adhesive. Accordingly, refractive index matching can be performed so that light reflection at the interface between the internal tissue is decreased, enabling an increase in observation performance.

Moreover, by bringing the face applied with the adhesive into close contact with the epidermis of the experimental animal, the observation window member can be easily attached to the epidermis without a gap. Therefore it becomes possible to prevent bacterial invasion into the internal tissue from the outside, and to prevent inflammation of the internal tissue caused by the bacterial invasion.

In the above aspect of the invention, preferably the observation window member is made from a material having oxygen permeability.

According to this structure of the invention, even if the observation window member is arranged attached to the internal tissue of the experimental animal, oxygen can be supplied to the surface of the internal tissue by permeating through the observation window member. Therefore the internal tissue can be maintained in a healthy condition for a longer term.

In the above aspect of the invention, preferably the observation window member is made from a material having an equivalent refractive index to water.

According to this structure of the invention, since body fluid contained in the internal tissue has an equivalent refractive index to water, by setting the refractive index of the observation window member to also be the same, it becomes possible to reduce light refraction in unexpected directions during the optical observation, or light reflection at the interface between the observation window member and the internal tissue, thus enabling highly accurate observation.

In the above aspect of the invention, preferably the observation window member is made from a material comprising a copolymer material.

According to this structure of the invention, the properties of a plurality of materials can be balanced to constitute an appropriate observation window member.

In the above aspect of the invention, preferably the observation window member is made from a material comprising a member having a refractive index of 1.5 or more, a surface of the member being coated with hydroxyacrylate.

The refractive index of an acrylate may be adjusted in a range of 1.45 to 1.59 by selecting the element to be combined. Since the refractive index of hydroxyacrylate is 1.45, light reflection at the interface between the observation window member and the internal tissue can be decreased. Furthermore, since the hydroxyacrylate is hydrophilic, there is the advantage that blood is hardly coagulated at the interface. Accordingly, observation performance can be increased and high visibility can be maintained while preventing blood coagulation even for long observation.

In the above aspect of the invention, the observation window member may be made from a material which contains a silicon rubber or an acrylic having a refractive index n of 1.45<n<1.5 and having the surface coated with a fluoropolymer.

Since the refractive index of fluoropolymers is 1.4 or less, if the surface is coated with a fluoropolymer by any method such as polymerization, light reflection at the interface with the internal tissue can be decreased. Moreover, since the fluoropolymers are hydrophobic, proteins can be kept from being adhered to the surface of the observation window member, and calcium can be also kept from being deposited thereon.

In the above aspect of the invention, it is effective if the observation window member has scale marks.

When observing the internal tissue, these can be observed using the scale marks on the observation window member as an index. Therefore, the size of the observation object in the internal tissue can be easily examined during the observation.

In the above aspect of the invention, preferably the observation window member has a filtering function which passes light having the necessary wavelength for observation, and blocks light having other wavelengths.

According to this structure of the invention, it becomes possible to extract only light having the necessary wavelength for observation, enabling noise to be reduced and observation accuracy to be increased.

Another aspect of the present invention is an experimental animal having an observation window, having the above mentioned observation window attached to an area where the epidermis has been removed.

According to this aspect of the invention, by using the animal in an observation which requires constant observation conditions to be maintained for a long term, highly accurate observation results can be obtained.

Yet another aspect of the present invention provides a method of treating an experimental animal, the method comprising: removing the epidermis of the experimental animal, and attaching the aforementioned observation window member to the area of the experimental animal where the epidermis has been removed.

According to this aspect of the invention, an experimental animal having the transparent observation window member in plate or membrane shape which is in close contact with the internal tissue, such that the internal tissue which can be observed from the outside through the observation window member, can be obtained.

According to the present invention, an effect is demonstrated such that the internal tissue can be easily observed while maintaining the experimental animal in a healthy condition for a long term. As a result, an effect is demonstrated such that it is possible to examine progress after drug dosage or after treatment, which requires continuous observation over a long term.

BRIEF DESCRIPTION OF THE SEVERAL VIEWS OF THE DRAWINGS

FIG. 1A and FIG. 1B show an observation window member according to an embodiment of the present invention, FIG. 1A being a front view and FIG. 1B being a transverse sectional view.

FIG. 2 shows a state where the epidermis of an experimental animal is peeled off, when attaching the observation window member shown in FIG. 1A and FIG. 1B.

FIG. 3 shows a state where the observation window member is attached to the abdomen of the experimental animal of which the epidermis was peeled off in FIG. 2.

FIG. 4 is a schematic diagram showing an observation apparatus for observing the experimental animal in FIG. 3.

FIG. 5 shows a state where three observation window members are attached to the abdomen of the experimental animal.

FIG. 6 shows a state where two observation window members are attached to the back of the experimental animal.

FIG. 7 shows a state where the main body of the observation apparatus of FIG. 4 is inclined.

FIG. 8 is a modified example of the observation window member of the present invention, being a transverse sectional view showing the observation window member adhered to the internal tissue by an adhesive.

FIG. 9 is a modified example of the observation window member of the present invention, being a transverse sectional view showing the observation window member adhered to the internal tissue by an adhesive.

DETAILED DESCRIPTION OF THE INVENTION

Hereunder is a description of an embodiment of an observation window member according to the present invention and an experimental animal furnished therewith, with reference to the drawings.

An observation window member 1 according to the present embodiment is described in the case where it is applied to a diseased model mouse which is assumed as an' experimental animal 2.

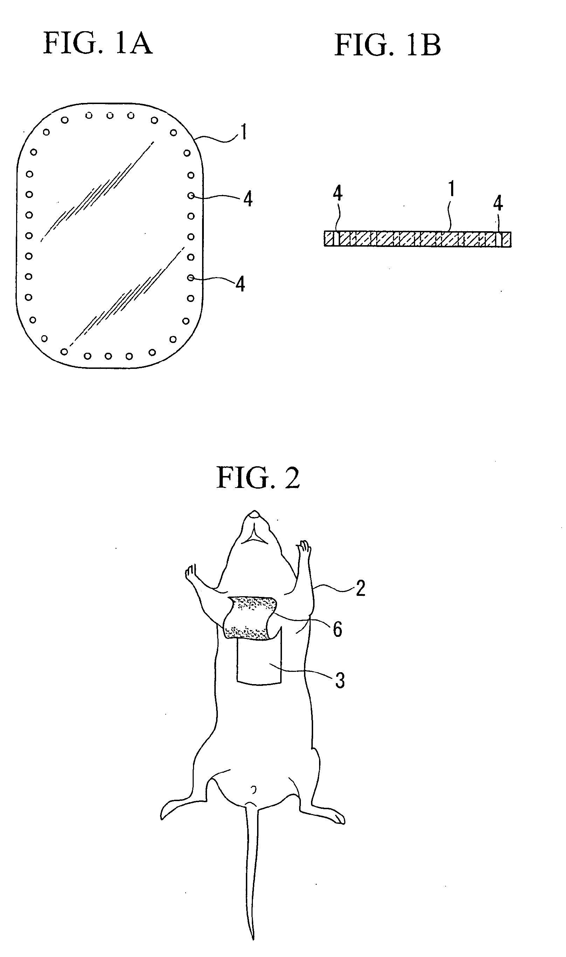

As shown in FIG. 1A and FIG. 1B, the observation window member 1 according to the present embodiment is a plate member made from an optically transparent material.

Examples of the material of the observation window member 1 include materials containing a plastic, a silicone, or an acrylic, more specifically, materials containing silk fibroin or an aromatic vinyl compound, materials using butylacrylate, materials containing a methacrylate such as glycerol methacrylate, hydroxyethyl methacrylate, FMA, or SMA, materials using a silicon rubber or methylsiloxane, materials using a fluoropolymer or a perfluoro compound, materials using N-vinylpyrrolidone or dimethylacrylamide, and materials containing collagen.

Moreover, the material of the observation window member 1 may be polymer materials using the above materials, which include for example, materials containing a graft polymer of silicon and either one of hydroxyethyl methacrylate and N-vinylpyrrolidone, and materials containing a polymer of a polysaccharide and an acrylic derivative.

When these materials are employed, since for example aromatic vinyl compounds have superior transparency, flexibility, and proper elasticity, the usage of a material containing such a compound as the observation window member can increase the closeness of contact with the internal tissue and the visibility of the internal tissue from the outside.

Since silk fibroin is superior in transparency, an observation window member having high visibility can be constructed.

Since materials containing butylacrylate have appropriate strength and durability, the observation window member can be kept from being damaged during the observation.

Since FMA and SMA are superior in the gas permeability, the internal tissue can be kept in a healthy condition for a long term.

If the surface of the observation window member is covered with a material such as collagen which has high bioadhesiveness, the adhesiveness of the observation window member to the internal tissue can be increased.

Since silicon rubbers, fluoropolymers and perfluoro compounds are hydrophobic or non-water-retaining materials, if a material containing such is used for the observation window member, internal substances such as proteins are hardly adhered to the surface of the observation window member, so that the transparency can be easily maintained. Moreover, since they are hydrophobic, calcium is hardly deposited so that the observation window member can be kept from being hardened, and the platelets can be kept from being damaged. Furthermore, these amorphous materials have high transparency so that visibility can be increased. Since silicon rubber is superior in gas permeability, the internal tissue can be kept in a healthy condition. Specifically, polydimethylsiloxane, which is one type of silicon rubber is superior in biocompatibility.

The graft polymers of silicon and either one of hydroxyethyl methacrylate and N-vinylpyrrolidone, the dimethylacrylamide, and the glycerol methacrylate are hydrophilic or water-retaining materials. If a material containing such is used for the observation window member, blood is hardly coagulated on the surface of the observation window member, and compatibility with the internal tissue is excellent, so that the transparency can be easily maintained. Specifically, glycerol methacrylate and hydroxyethyl methacrylate are highly flexible so that close contact with the internal tissue can be maintained even if the experimental animal moves.

Examples of polysaccharides include agar, arabian gum, pectin, tragacanth gum, galactomannan, cellulose, and xylitol. By polymerizing such a polysaccharide and an acrylic derivative, an observation window member having superior biocompatibility can be constructed. Moreover, not limited to this, by using as the observation window member a copolymer material in which the aforementioned plurality of materials are used, the abovementioned plurality of properties can be properly balanced. Specifically, it becomes possible to balance the strength and the gas permeability, or to balance the flexibility and the transparency, so that more appropriate materials can be obtained for the observation window member.

Furthermore, a polyurethane elastomer may be employed as the biocompatible material.

All of these materials are biocompatible so that they can be in close contact with the internal tissue 3 without generating any rejection reaction such as down growth in the internal tissue 3 in contact therewith. Therefore, they can protect the internal tissue 3 by being in close contact with the internal tissue 3, becoming a part of the skin.

The observation window member 1 according to the present embodiment has a plurality of through holes (attachment section) 4 at intervals along the periphery. The through holes 4 have diameters to allow a thread 5 for stitching to the experimental animal 2 to pass therethrough. The through holes are provided at appropriate intervals, for example intervals of about 1 mm to 3 mm.

The thickness of the observation window member 1 may be for example, about 0.1 mm to 2 mm. If a flexible material is used, the material can be curved to match the curvature of the part being attached, so as to be in close contact with this part.

Next is a description of the case of attaching the observation window member 1 according to the present embodiment, which is constructed in the above manner.

In attaching the observation window member 1 according to the present embodiment to the experimental animal 2 which is a diseased model mouse for example, as shown in FIG. 2, the part to be attached to, for example the abdominal epidermis 6, is peeled off by operation.

Once the internal tissue 3 are exposed by peeling off the epidermis 6, the observation window member 1 according to the present embodiment is brought into close contact with the surface of the exposed internal tissue 3. By making the observation window member 1 from a flexible material as described above, it can be attached without a gap between it and the internal tissue 3.

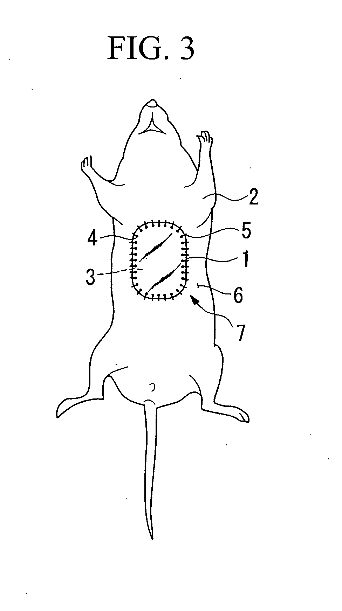

Then, as shown in FIG. 3, the observation window member 1 in close contact with the internal tissue 3 of the diseased model mouse 2 of which the epidermis 6 was peeled off, is stitched to the epidermis 6 which is arranged around the exposed part of the internal tissue 3. Since the plurality of through holes 4 are arranged on the periphery of the observation window member 1, the thread 5 can easily pass through by piercing the through holes 4 with a needle, and the stitching operation can be easily performed.

Accordingly, the experimental animal 2 comprising an observation window 7 made up by exposing the internal tissue 3 and covering the surface of the internal tissue 3 with the transparent observation window member 1, is constructed.

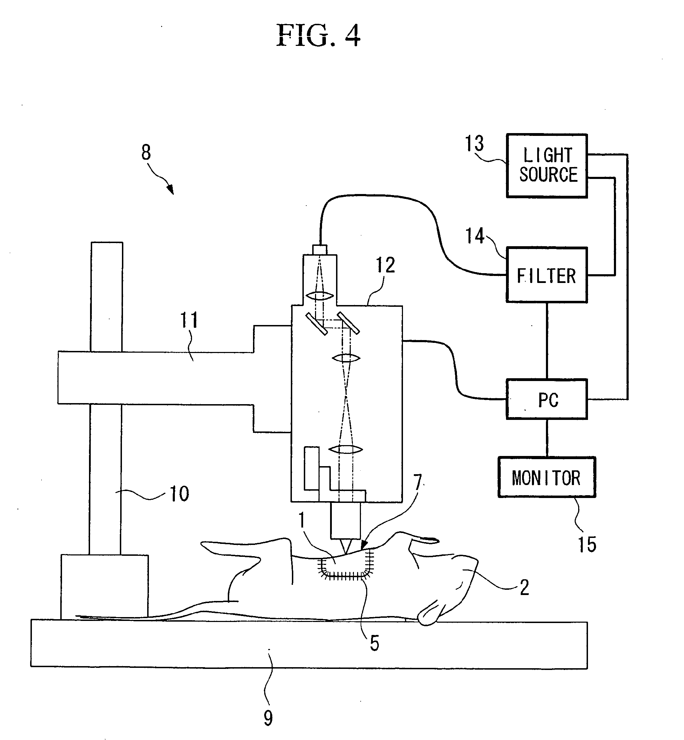

The experimental animal 2 constructed in this manner is observed by an observation apparatus 8 shown in FIG. 4 for example.

That is, this observation apparatus 8 comprises: a stage 9 for mounting the experimental animal 2, a vertical shaft 10 which is fixed to the stage 9, an arm 11 which is attached to the vertical shaft so as to be movable in the vertical direction, an apparatus main body 12 which is attached to the end of the arm 11, a light source 13 which supplies visible light or infrared light to the apparatus main body 12, a filter 14 which extracts light having the necessary wavelength for observation from the light emitted from the light source 13, a computer PC which controls the light source 13, the filter 14 and the apparatus main body 12, and a monitor 15 which is connected to the computer PC.

A fastening device for fixing the experimental animal 2, is provided on the stage 9.

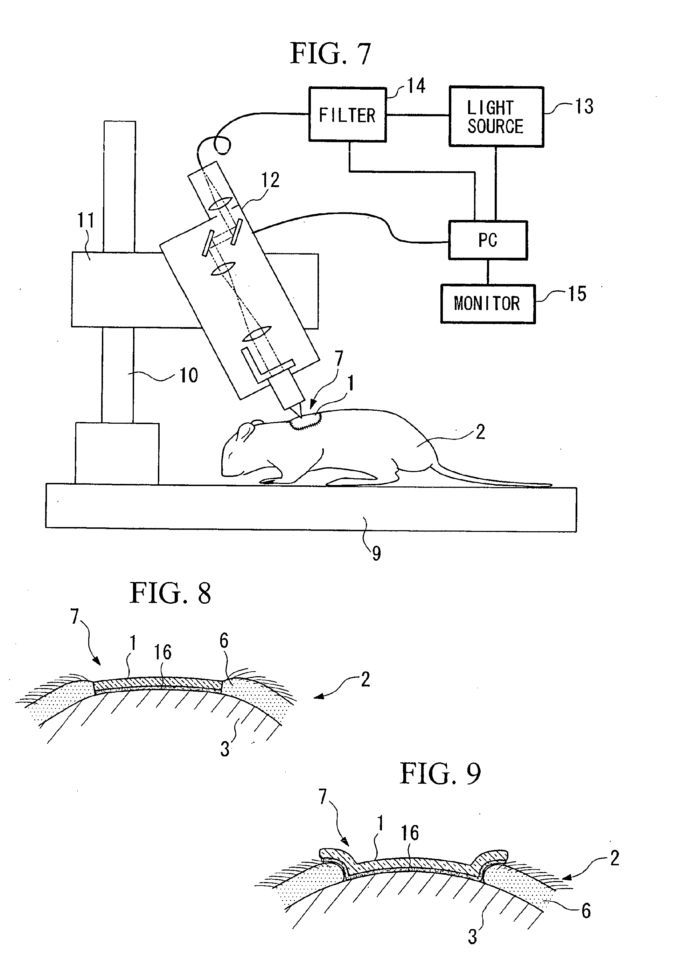

The arm 11 can move vertically along the vertical shaft 10, to match with the size of the experimental animal 2 mounted on the stage 9. Moreover, as shown in FIG. 7, the angle of the apparatus main body 12 at the end of the arm 11 can be adjusted to match with the angle of inclination of the part being observed of the experimental animal 2.

The apparatus main body 12 is constructed such that light emitted from the light source 13 and passing through the filter 14, passes through the observation window 7 of the experimental animal 2 and is incident on the internal tissue 3, and the light reflected from the internal tissue 3 is detected. The detected light is sent to the computer PC so that it can be displayed via a connected monitor 15, and is subjected to appropriate image processing so that it can be recorded as electronic information. A confocal optical system or other optional optical system may be employed for the optical system in the apparatus main body 12.

Specifically, the methods of observing the internal tissue of the experimental animal include bright field observation, fluorescent observation, two-photon absorption observation, and the like. In the fluorescent observation, self-fluorescence in the internal tissue of the experimental animal 2, or luminescent proteins or fluorochromes which were bound with the proteins in vivo are observed. At this time, if self-fluorescence exists in the observation window member, in the case where it is overlapped with the fluorescence from the luminescent proteins such as GFP, DsRed, or RFP in vivo, the luminescent proteins are hardly observed. Therefore, self-fluorescence of the material used for the observation window member is preferably minimal, or has a wavelength which does not overlap with the fluorescence from the luminescent proteins.

According to the observation window member 1, and the experimental animal 2 comprising the observation window 7, of the present embodiment, the internal tissue 3 which was exposed by peeling off the epidermis 6 for the purpose of observation, is covered with the observation window member 1 so that the internal tissue 3 can be observed while being protected. Therefore, bacterial infection or inflammation through the surface of the exposed internal tissue 3 can be kept from being generated so that the experimental animal 2 can be kept in a healthy condition for a long term.

That is, according to the observation window member 1 and the experimental animal 2 comprising the observation window 7, of the present embodiment, even in the case where long term continuous observation is required, there is the effect that the experimental animal 2 can be kept from being debilitated due to other factors, and can be observed with high accuracy. Moreover, there is no limitation on the number of times for observation, and it can be repeatedly observed at any time, so that there is the effect that a more detailed observation can be performed.

Furthermore, since the observation window 7 is made from a material having optically high transparency, the internal tissue 3 can be directly observed from the outside. Therefore, even in the case where GFP is used for the diseased model mouse 2, the fluorescence can be detected more clearly from the outside, increasing the observation accuracy.

A diseased model mouse was used and described as an example of the experimental animal 2 for application of the observation window member 1 according to the present invention. However it is not limited to this and needless to say the observation window member 1 may be applied to other experimental animals 2 besides mice.

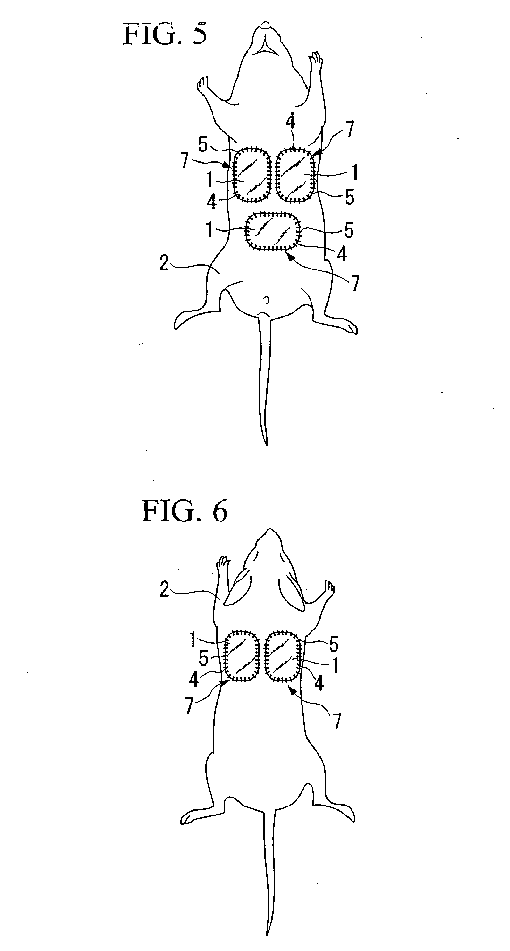

Moreover, one part of the abdominal epidermis 6 was peeled off to provide the observation window 7. However, instead of this, as shown in FIG. 5, a plurality of observation windows 7 may be provided corresponding to the parts being observed. Accordingly, the epidermis 6 can be kept from being peeled off in a wide area so as to suppress damage to the experimental animal 2.

Moreover, as shown in FIG. 6, the observation windows 7 may be provided with the epidermis 6 on the back peeled off instead of on the abdomen.

In the present embodiment, the through holes 4 for stitching were provided in the periphery of the observation window member 1. However, instead of this, as shown in FIG. 8, the observation window member 1 may be adhered to the internal tissue 3 which is exposed by peeling off the epidermis 6, using an adhesive 16. In this case, the adhesive 16 is preferably a biocompatible material having optically high transparency, for example fibrin glue adhesive or the like.

By having such a construction the observation window member 1 can be easily attached to the internal tissue 3 without a gap.

Alternatively as shown in FIG. 9, an observation window member 1 which is larger than the area exposed by peeling off the epidermis 6, may be adhered to the epidermis 6 of the experimental animal 2 by the adhesive 16. In this case, the area of application of the adhesive 16 to the observation window member 1 may be only the part in contact with the epidermis 6, or both the part in contact with the epidermis 6 and the part in contact with the internal tissue 3. By having such a construction, the observation window member 1 can be easily attached to the internal tissue 3 without a gap, and it becomes possible to prevent bacterial invasion into the internal tissue 3 from the outside, and inflammation of the internal tissue 3 caused by the bacterial invasion.

Moreover, the observation window member 1 may be constructed as a seal type which is formed in a plate or membrane shape, with one face applied with the above adhesive 16. By having such a construction, the experimental animal 2 having the observation windows 7 can be produced more easily than with stitching.

Moreover, material having oxygen permeability is preferably employed for the above observation window member 1. Siloxanylmethacrylate or fluoromethacrylate is preferably used in order to have oxygen permeability.

Accordingly, oxygen can be supplied to the covered internal tissue 3 through the observation window member 1, so that the internal tissue 3 can be kept in a healthy condition for a longer term.

Moreover, a material having an equivalent refractive index to water, for example, a fluoropolymer, specifically CYTOP (trademark) made by Asahi Glass Co., Ltd., may be employed for the observation window member 1. Fluoropolymers have a feature in that the reflection generated at the interface with water can be suppressed, since the refractive index is low at 1.35 or less.

Since the internal tissue 3 is filled with body fluid having an equivalent refractive index to water, by making the observation window member 1 from a material having a refractive index which is equivalent to water or less, it becomes possible to reduce the generation of noise caused by light reflection at the interface, enabling highly accurate observation to be performed.

CYTOP (trademark) is one type of fluoropolymer, being an amorphous fluoropolymer of the perfluoro type. Since this material is a fluoropolymer, it has the quality as described above in that proteins are hardly adhered, and since it is amorphous, the material has high transparency. Moreover, since the refractive index is 1.34 which is approximately the same value as the refractive index of water, a water immersion object lens may be used. Furthermore, since there is no self-fluorescence, there is the advantage that it is possible to prevent a decrease in observation accuracy due to luminescent proteins such as GFP, DsRed or RFP overlapping with the self-fluorescence of the observation window member 1.

Moreover, when the observation window member 1 is adhered by the adhesive 16, the adhesive is preferably made from a material having a refractive index which is equivalent to or less than that of water.

Alternatively for the adhesive 16, a material having a refractive index higher than but close to the refractive index of water, for example a copolymer material using a fluorine containing material and a silicone containing material may be used.

Moreover, the adhesive 16 may be applied over the whole surface of the observation window member 1, or may be applied to only the periphery. If the adhesive 16 is applied within the observation area of the observation window member 1, a material having an intermediate refractive index between the refractive index of the observation window member 1 and the refractive index of water is preferably employed for the adhesive 16. If the adhesive 16 is not applied within the observation area of the observation window member 1, the aforementioned material having an intermediate refractive index is preferably coated on the surface of the observation window member 1 which is to be in contact with the internal tissue 3.

Accordingly, refractive index matching can be achieved without abrupt change in the refractive index. Therefore there is the effect that light reflection at the interface between the observation window member 1 and the internal tissue 3 can be decreased, and observation accuracy can thus be increased.

Moreover, a material having a refractive index of 1.5 or more with a surface coated with hydroxyacrylate may be employed for the observation window member 1.

The refractive index n of an acrylate may be adjusted in a range of $1.45 < n < 1.59$ by selecting the element to be combined. For example, an observation window member 1 having a surface coated with hydroxyacrylate by a method such as polymerization may be employed. Since the refractive index of hydroxyacrylate is n=1.45, light reflection at the interface with the internal tissue 3 in contact therewith can be decreased.

Furthermore, since the hydroxyacrylate is hydrophilic, there is the advantage that blood is hardly coagulated on the surface in contact with the internal tissue 3. Accordingly, a reduction in visibility of the internal tissue 3 through the observation window member 1 can be prevented.

Alternatively, a material having a refractive index n of $1.45 < n < 1.5$ which contains a silicon rubber or an acrylic and which has a surface coated with a fluoropolymer may be employed for the observation window member 1.

Since the refractive index n of fluoropolymers is 1.4 or less, by coating the surface with a fluoropolymer by any method such as polymerization, light reflection at the interface with the internal tissue 3 in contact therewith can be decreased. Moreover, since fluoropolymers are hydrophobic, proteins can be kept from being adhered to the surface of the observation window member 1 which is in close contact with the internal tissue 3, and calcium can be also kept from being deposited thereon, so that there is the effect that visibility can be maintained in a manner similar to the above.

Moreover, as an alternative to the observation window member as described above made from a single material and having the surface coated, an observation window member having a part functioning as a window and also having an adhesive section, the part functioning as a window and the adhesive section being made from a different material, may be employed for the observation window member 1.

If the adhesive 16 is not applied within the observation area of the observation window member 1, a material having low transparency may be used for the adhesive 16.

The observation window member according to the present embodiment is made from a flexible material. However it is not limited to this, and may be made from an elastic material. In this case, even if the experimental animal moves violently or bends and stretches, the observation window member can also be expanded and contracted to match the movement. Therefore, the epidermis around the observation window member will not be suddenly tensioned, so that the epidermis can be kept from being damaged, and the stress on the experimental animal can be decreased.

Moreover, scale marks (not shown) may be put on the whole surface or a part of the observation window member 1. Accordingly, the observation image, particularly the image displayed by the monitor, can be displayed together with the scale marks, so that the observer can conveniently see the size of the observation object at a glance.

**Claims**

1. An observation window member made from an optically transparent and biocompatible material formed in a plate or membrane shape to be in close contact with internal tissue of an experimental animal in an area where the epidermis has been removed.

2. An observation window member according to claim 1, made from a flexible material.

3. An observation window member according to claim 1, having an attachment section for the experimental animal.

4. An observation window member according to claim 3, wherein the attachment section is made of a plurality of through holes which are provided at intervals along the periphery and through which a thread member for stitching to the epidermis of the experimental animal can pass.

5. An observation window member according to claim 3, wherein the attachment section comprises an adhesive applied on one face.

6. An observation window member according to claim 1, made from a material having oxygen permeability.

7. An observation window member according to claim 1, made from a material having an equivalent refractive index to water.

8. An observation window member according to claim 1, made from a material comprising a copolymer material.

9. An observation window member according to claim 1, made from a material comprising a member having a refractive index of 1.5 or more, a surface of the member being coated with hydroxyacrylate.

10. An observation window member according to claim 1, made from a material which contains a silicon rubber or an acrylic having a refractive index n satisfying the following expression, and having the surface coated with a fluoropolymer:

$$1.45 < n < 1.5.$$

11. An observation window member according to claim 1, having scale marks.

12. An observation window member according to claim 1, having a filtering function which passes light having the necessary wavelength for observation, and blocks light having other wavelengths.

13. An experimental animal having an observation window, having the observation window member of claim 1 attached to an area where the epidermis has been removed.

14. A method of treating an experimental animal, the method comprising: removing the epidermis of the experimental animal, and attaching the observation window member of claim 1 to the area of the experimental animal where the epidermis has been removed.

FIG. 1A

FIG. 1B

FIG. 2

# FIG. 3

# FIG. 4

FIG. 5

FIG. 6

## FIG. 7

## FIG. 8

## FIG. 9

**European Patent**
**Office**

**PARTIAL EUROPEAN SEARCH REPORT**

**Application Number**

which under Rule 45 of the European Patent Convention EP 04 02 5831
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CIANCIO SEBASTIAN J., COBURN MICHAEL, HORNSBY PETER J.: "Cutaneous Window for in Vivo Observation of Organs and Angiogenesis" JOURNAL OF SURGICAL RESEARCH, vol. 92, 2000, pages 228-232, XP002319384 * abstract; figures 1,2 * ----- | 1-14 | A61L31/04 |
| A | US 2002/072657 A1 (BOUSQUET GERALD G ET AL) 13 June 2002 (2002-06-13) * figures * * claims * ----- | 1-14 | |
| A | WO 03/022042 A (ANTICANCER, INC) 20 March 2003 (2003-03-20) * claims * ----- | 1-14 | |

| | TECHNICAL FIELDS SEARCHED (Int.Cl.7) |
|---|---|
| | A61B A61L |

### INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 28 February 2005 | Thornton, S |

**European Patent**
**Office**

**INCOMPLETE SEARCH**
**SHEET C**

**Application Number**

EP 04 02 5831

Although claims 14 is directed to a method of treatment of the human/animal body (Article 52(4) EPC), the search has been carried out and based on the alleged effects of the composition.

　　　　-----

Claim(s) not searched:
　　　　13

Reason for the limitation of the search (non-patentable invention(s)):

Article 52 (4) EPC - Method for treatment of the human or animal body by surgery
Article 53 (b) EPC - Animal variety

# EP 1 530 977 A1

## ANNEX TO THE EUROPEAN SEARCH REPORT
## ON EUROPEAN PATENT APPLICATION NO.

EP 04 02 5831

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2005

| Patent document cited in search report | | Publication date | Patent family member(s) | | Publication date |
|---|---|---|---|---|---|
| US 2002072657 | A1 | 13-06-2002 | NONE | | |
| WO 03022042 | A | 20-03-2003 | CA | 2460049 A1 | 20-03-2003 |
| | | | EP | 1435774 A1 | 14-07-2004 |
| | | | WO | 03022042 A1 | 20-03-2003 |
| | | | US | 2003088885 A1 | 08-05-2003 |

EPO FORM P0459

For more details about this annex : see Official Journal of the European Patent Office, No. 12/82

16